# EUROPÄISCHE PATENTSCHRIFT

(11) **EP 2 558 473 B1**
(45) Veröffentlichungstag und Bekanntmachung des Hinweises auf die Patenterteilung: **07.09.2016**
(21) Anmeldenummer: 11712882.7
(22) Anmeldetag: 08.04.2011
(51) Int. Cl.: C07D 495/14, C07D 207/456

(54) **VERFAHREN ZUR HERSTELLUNG VON DITHIIN-TETRACARBOXIMIDEN**
METHOD FOR PRODUCING DITHIIN TETRACARBOXIMIDES
PROCÉDÉ DE FABRICATION DE DITHIINE-TÉTRACARBOXIMIDES

(30) Priorität: 16.04.2010 US 325074 P; 14.04.2010 EP 10159899
(43) Veröffentlichungstag der Anmeldung: 20.02.2013
(73) Patentinhaber: Bayer Intellectual Property GmbH, 40789 Monheim am Rhein (DE)
(72) Erfinder: HIMMLER, Thomas, 51519 Odenthal (DE); ETZEL, Winfried, 42799 Leichlingen (DE); VOLZ, Frank, 50825 Köln (DE)
(74) Vertreter: BIP Patents
(86) Internationale Anmeldenummer: PCT/EP2011/055512
(87) Internationale Veröffentlichungsnummer: WO 2011/128263

(56) Entgegenhaltungen:
- VALLA, ALAIN ET AL: "Atypical oxidation reaction by thionyl chloride. Easy two-step synthesis of N-alkyl-1,4-dithiines", SYNTHETIC COMMUNICATIONS , 36(23), 3591-3597 CODEN: SYNCAV; ISSN: 0039-7911, 2006, XP002599895, in der Anmeldung erwähnt
- ZENTZ ET AL: "Syntheses, in vitro antibacterial and antifungal activities of a series of N-alkyl, 1,4-dithiines", FARMACO, SOCIETA CHIMICA ITALIANA, PAVIA, IT LNKD- DOI:10.1016/J.FARMAC.2005.06.015, Bd. 60, Nr. 11-12, 1. November 2005 (2005-11-01), Seiten 944-947, XP005151567, ISSN: 0014-827X in der Anmeldung erwähnt
- YUN, JI YEONG ET AL: "Quantitative regioselective Diels-Alder reaction of an unsymmetrical 1,4-dithiin and anthracene through heterogeneous solid state conversion", DYES AND PIGMENTS , 83(2), 262-265 CODEN: DYPIDX; ISSN: 0143-7208, 2009, XP002599896,
- GUELTEN, SIRIN: "The synthesis and characterization of solvatochromic maleimide-fused N-allyl- and N-alkyl-substituted 1,4-dithiines and Diels-Alder reactions with anthracene", JOURNAL OF HETEROCYCLIC CHEMISTRY , 47(1), 188-193 CODEN: JHTCAD; ISSN: 1943-5193, 8. Januar 2010 (2010-01-08), XP002599897,

## Beschreibung

Die vorliegende Erfindung betrifft ein neues Verfahren zur Herstellung von Dithiin-tetracarboximiden.

Dithiin-tetracarboximide als solche sind bereits bekannt. Ebenso ist bekannt, dass diese Dithiin-tetracarb-oximide als Anthelminthika gegen innere Parasiten von Tieren, insbesondere Nematoden, verwendet werden können und insektizide Wirkung aufweisen (vgl. US 3,364,229). Außerdem ist bekannt, dass bestimmte Dithiin-tetracarboximide antibakterielle Wirkung besitzen und gegen Erreger von Mykosen beim Menschen eine gewisse Wirkung aufweisen (vgl. II Farmaco 2005, 60, 944-947). Weiterhin ist bekannt, dass Dithiin-tetracarboximide als Pigmente in elektrophotographischen Photorezeptoren oder als Farbstoffe in Lacken und Polymeren eingesetzt werden können (vgl. JP-A 10-251265, PL-B 143804).

Dithiin-tetracarboximide der Formel (I) 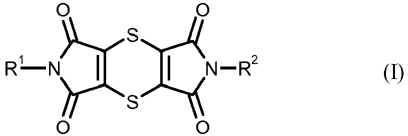 in welcher
R¹ und R² gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
R³ für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
R⁴ für Hydroxy, C₁-C₄-Alkyl oder C₁-C₄-Alkoxy steht,
können auf verschiedene bekannte Weisen hergestellt werden.

Beispielsweise wird in einem Verfahren (vgl. US 3,364,229; Chem. Ber. 1967, 100, 1559-1570) in einer ersten Stufe Dichlormaleinsäureanhydrid der Formel (II) mit einem Amin der Formel (III) gegebenenfalls in Gegenwart eines Verdünndungsmittels umgesetzt. Anschließend werden dann die so erhaltenen Dichlormaleinsäureimide der Formel (IV) mit einer Schwefelverbindung (z.B. Schwefelwasserstoff oder Thioharnstoff) umgesetzt. Die Herstellung der Dithiin-tetracarboximide der Formel (I) nach diesem Verfahren kann durch das folgende Schema veranschaulicht werden:

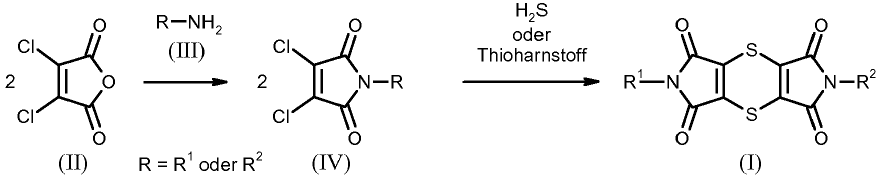

Dieses Verfahren hat den Nachteil, dass beispielsweise der Umgang mit dem hochgiftigen Schwefelwasserstoffgas technisch sehr schwierig und aufwendig ist. Bei der Verwendung von Thioharnstoff werden außer dem Zielprodukt unerwünschte Nebenprodukte erhalten, die nur sehr schwierig zu entfernen sind und die erreichbaren Ausbeuten verschlechtern (vgl. J. Heterocycl. Chem. 1988, 25, 901-906).

In einem weiteren bekannt gewordenen Verfahren (vgl. Synthetic Communications 2006, 36, 3591-3597) wird in einer ersten Stufe Bernsteinsäureanhydrid der Formel (V) mit einem Amin der Formel (III) gegebenenfalls in Gegenwart eines Verdünndungsmittels umgesetzt. Anschließend werden die so erhaltenen Bernsteinsäuremonoamide der Formel (VI) 6 Stunden mit einem großen Überschuss an Thionylchlorid in Gegenwart von Dioxan als Verdünnungsmittel bei Raumtemperatur umgesetzt, wobei in einer Abfolge zahlreicher Reaktionsschritte schließlich die Dithiin-tetracarboximide der Formel (I) erhalten werden. Die Dithiin-tetracarboximide werden wahlweise direkt aus dem Reaktionsgemisch oder nach Versetzen mit Wasser durch Filtration isoliert. Je nach Reaktionsbedingungen (Verdünnungsmittel) und Art der Reste R können unter Umständen die Dithiin-diisoimide der Formel (VII) isoliert werden, bevor man sie in die Dithiin-tetracarboximide der Formel (I) überführt. Diese Herstellungsmethode der Dithiin-tetracarboximide der Formel (I) kann durch das folgende Schema veranschaulicht werden:

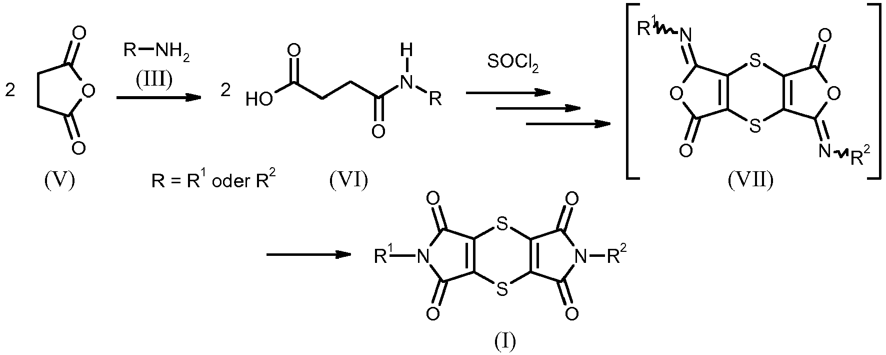

Nachteilig an diesem Verfahren sind die lange Reaktionszeit sowie das Ergebnis, dass entweder die erhaltenen Ausbeuten in der Regel etwa 30-40% der Theorie nicht überschreiten oder aber die Reinheiten der isolierten Produkte ungenügend sind (s. Vergleichsbeispiele). Außerdem ist bei einer wässrigen Aufarbeitung des Reaktionsgemisches nachteilig, dass dabei große Mengen Thionylchlorid vernichtet werden; die entstehenden Gase (SO₂ und HCl) müssen entsorgt werden. Ebenfalls nachteilig ist der Umstand, dass erfahrungsgemäß (s. Vergleichsbeispiele) das Produkt nicht in einer Fraktion erhalten wird. Vielmehr ist es häufig so, dass nach einer ersten Produktisolierung durch Filtration aus dem Filtrat nach längerem Stehen (beispielsweise über Nacht), weiteres Produkt ausfällt, das erneut durch Filtration isoliert werden muss. Zuweilen muss dieser Vorgang nochmals durchgeführt werden. Diese Arbeitsweise ist sehr umständlich und zeitraubend.

Weiterhin ist bekann, dass man Dithiin-tetracarboximide erhält, indem man N-substituierte Bernsteinsäureamide in trockenem 1,4-Dioxan löst und anschließend mit Thionylchlorid versetzt. Anschließend wird das Reaktionsgemisch erwärmt und die Lösung wird in vacuo eingeengt und über Säulenchromatographie aufgetrennt und gereinigt (vgl. J. Heterocycl. Chem. 2010, 47, 188-193).

Es besteht demnach weiterhin Bedarf an einem technisch einfachen und ökonomischen Herstellverfahren für Dithiin-tetracarboximide der Formel (I).

Es wurde ein neues Verfahren zum Herstellen von Dithiin-tetracarboximide der allgemeinen Formel (I) 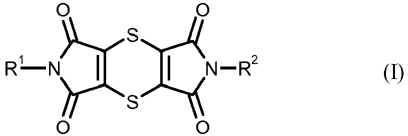 in welcher R¹ und R² die oben angegebenen Bedeutungen haben,
gefunden, dadurch gekennzeichnet, dass man
in einer ersten Stufe Bernsteinsäuremonoamide der Formel (VI) 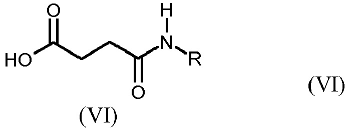 in welcher R für R¹ oder R² steht,
mit einem Überschuss an Thionylchlorid gegebenenfalls in Gegenwart eines Verdünnungsmittels umsetzt, anschließend den Überschuss an Thionylchlorid entfernt und das so erhaltene Produktgemisch in einer zweiten Stufe in einem organischen Lösungsmittel in die Dithiin-tetracarboximide der Formel (I) überführt.

Auf diese Weise können die Dithiin-tetracarboximide der Formel (I) in höherer Ausbeute, kürzerer Zeit und besserer Reinheit erhalten werden.

Das im ersten Schritt des erfindungsgemäßen Verfahrens erhaltene Produktgemisch enthält auch bereits Dithiin-tetracarboximide der Formel (I), als Hauptkomponenten jedoch Polysulfide der Formel (IX), 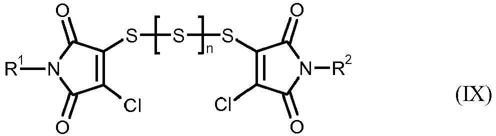 sowie, je nach Aufarbeitungsmethode, auch Thiosulfonsäurederivate der Formel (VIII)

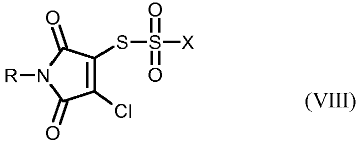

Die Thiosulfonsäurederivate der allgemeinen Formel (VIII) und die Polysulfide der allgemeinen Formel (IX) sind neu und ebenfalls Gegenstand der vorliegenden Erfindung.

In den Thiosulfonsäurederivaten der allgemeinen Formel (VIII) steht R für die oben angegebenen Bedeutungen von R¹ bzw. R² und X für Chlor oder Hydroxy.

In den Polysulfiden der allgemeinen Formel (IX) stehen R¹ und R² für die oben angegebenen Bedeutungen und n für 0, 1 oder 2.

Verbindungen der allgemeinen Formel (VIII) erhält man neben anderen Produkten dann, wenn das Reaktionsgemisch nach der Umsetzung der Verbindungen der allgemeinen Formel (VI) mit Thionylchlorid eingeengt wird.

Verbindungen der allgemeinen Formel (IX) erhält man neben anderen Produkten dann, wenn man das Reaktionsgemisch nach der Umsetzung der Verbindungen der allgemeinen Formel (VI) mit Thionylchlorid einengt, in einem inerten, nicht mit Wasser mischbaren Lösungsmittel wie beispielsweise Methylenchlorid löst und bei Raumtemperatur mit Wasser ausschüttelt. Nach Abtrennen der organischen Phase, Trocknen und Einengen erhält man ein Gemisch, das neben Dithiin-tetracarboximiden der Formel (I) hauptsächlich Verbindungen der allgemeinen Formel (IX) enthält.

Das erfindungsgemäße Verfahren zur Herstellung der Dithiin-tetracarboximide der Formel (I) kann durch das folgende Schema veranschaulicht werden:

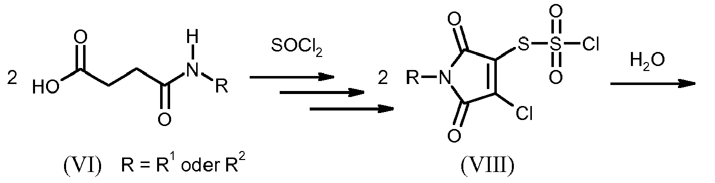

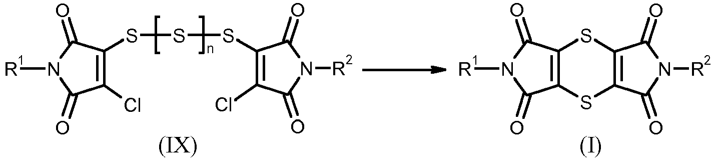

Die bei der Durchführung des erfindungsgemäßen Verfahrens als Ausgangstoffe eingesetzten Bernsteinsäuremonoamide sind durch die Formel (VI) allgemein definiert. R steht für die Bedeutungen von R¹ oder R².
- R¹ und R²: sind bevorzugt gleich oder verschieden und stehen bevorzugt für Wasserstoff, für gegebenenfalls einfach oder mehrfach durch Fluor, Chlor, Brom, -OR³, -COR⁴ substituiertes C₁-C₆-Alkyl, gegebenenfalls einfach oder mehrfach durch Chlor, Methyl oder Trifluormethyl substituiertes C₃-C₇-Cycloalkyl, für jeweils gegebenenfalls einfach oder mehrfach durch Fluor, Chlor, Brom, Methyl, Trifluormethyl, -COR⁴, Sulfonylamino substituiertes Phenyl oder Phenyl-(C₁-C₄-alkyl).
- R¹ und R²: sind besonders bevorzugt gleich oder verschieden und stehen besonders bevorzugt für Wasserstoff, für gegebenenfalls einfach oder mehrfach durch Fluor, Chlor, Hydroxy, Methoxy, Ethoxy, Methylcarbonyloxy, Carboxyl substituiertes C₁-C₄-Alkyl, gegebenenfalls einfach oder mehrfach durch Chlor, Methyl oder Trifluormethyl substituiertes C₃-C₇-Cycloalkyl, für jeweils gegebenenfalls einfach bis dreifach durch Fluor, Chlor, Brom, Methyl, Trifluormethyl, -COR⁴, Sulfonylamino substituiertes Phenyl, Benzyl, 1-Phenethyl, 2-Phenethyl oder 2-Methyl-2-phenethyl.
- R¹ und R²: sind ganz besonders bevorzugt gleich oder verschieden und stehen ganz besonders bevorzugt für Wasserstoff, Methyl, Ethyl, n-Propyl, Isopropyl, 2,2-Difluorethyl, 2,2,2-Trifluorethyl, jeweils gegebenenfalls durch Chlor, Methyl oder Trifluormethyl substituiertes Cyclopropyl oder Cyclohexyl.
- R¹ und R²: stehen insbesondere bevorzugt gleichzeitig für Methyl.
- R³: steht bevorzugt für Wasserstoff, Methyl, Ethyl, Methylcarbonyl, Ethylcarbonyl oder für gegebenenfalls einfach oder mehrfach durch Fluor, Chlor, Methyl, Ethyl, n-Propyl, Isopropyl oder Trifluormethyl substituiertes Phenyl.
- R³: steht besonders bevorzugt für Wasserstoff, Methyl, Methylcarbonyl oder für Phenyl.
- R⁴: steht bevorzugt für Hydroxy, Methyl, Ethyl, Methoxy oder Ethoxy.
- R⁴: steht besonders bevorzugt für Hydroxy oder Methoxy.
Besonders bevorzugt wird Bernsteinsäuremethylamid als Ausgangsstoff eingesetzt, wodurch man als Endprodukt die Verbindung (I-1) 2,6-Dimethyl-1H,5H-[1,4]dithiino[2,3-c:5,6-c']dipyrrol-1,3,5,7(2H,6H)-tetron erhält.

Setzt man Bernsteinsäuretertiärbutylamid als Ausgangsstoff ein, erhält man die Verbindung (I-2) 2,6-Di-tert-butyl-1H,5H-[1,4]dithiino[2,3-c:5,6-c']dipyrrol-1,3,5,7(2H,6H)-tetron als Endprodukt.

Setzt man Bernsteinsäurecyclohexylamid als Ausgangsstoff ein, erhält man die Verbindung (I-3) 2,6-Dicyclohexyl-1H,5H-[1,4]dithiino[2,3-c:5,6-c']dipyrrol-1,3,5,7(2H,6H)-tetron als Endprodukt.

Setzt man Bernsteinsäurepropylamid als Ausgangsstoff ein, erhält man die Verbindung (I-4) 2,6-Dipropyl-1H,5H-[1,4]dithiino[2,3-c:5,6-c']dipyrrol-1,3,5,7(2H,6H)-tetron als Endprodukt.

Als Zwischenprodukte erhält man besonders bevorzugt
(VIII-1) S-(4-Chlor-1-methyl-2,5-dioxo-2,5-dihydro-1H-pyrrol-3-yl)chlorothiosulfat (R = Me, X = Cl),
(IX-1) 3,3'-Trisulfan-1,3-diylbis(4-chlor-1-methyl-1H-pyrrol-2,5-dion) (R¹ = R² = Me, n= 1)
(IX-2) 3,3'-Disulfanediylbis(4-chlor-1-methyl-1H-pyrrol-2,5-dion) (R¹ = R² = Me, n = 0)
(IX-3) 3,3'-Disulfanediylbis(1-tert-butyl-4-chlor-1H-pyrrol-2,5-dion) (R¹ = R² = t-Bu, n = 0)
(IX-4) 3,3'-Trisulfan-1,3-diylbis(1-tert-butyl-4-chlor-1H-pyrrol-2,5-dion) (R¹ = R² = t-Bu, n = 1)
(IX-5) 3,3'-Trisulfan-1,3-diylbis(4-chlor-1-cyclohexyl-1H-pyrrol-2,5-dion) (R¹ = R² = Cyclohexyl , n = 1)

Die Menge an Thionylchlorid im ersten Schritt des erfindungsgemäßen Verfahrens liegt zwischen 2 und 100 Mol pro Mol Bernsteinsäuremonoamid der Formel (VI). Bevorzugt verwendet man zwischen 4 und 50 Mol, besonders bevorzugt Mengen zwischen 10 und 40 Mol pro Mol Bernsteinsäuremonoamid der Formel (VI).

Die Reaktionstemperatur im ersten Schritt des erfindungsgemäßen Verfahrens kann in weiten Grenzen variiert werden und liegt zwischen 0°C und 150°C. Zur Erzielung befriedigender Raum-Zeit-Ausbeuten wird man bevorzugt bei Temperaturen zwischen 20°C und 120°C arbeiten; besonders bevorzugt zwischen 30°C und 100°C.

Die Reaktionszeit im ersten Schritt des erfindungsgemäßen Verfahrens liegt zwischen 10 Minuten und 24 Stunden. Bevorzugt arbeitet man zwischen 30 Minuten und 6 Stunden, besonders bevorzugt zwischen 1 und 4 Stunden.

Der erste Schritt des erfindungsgemäßen Verfahrens kann gegebenenfalls in Gegenwart eines unter den Reaktionsbedingungen möglichst inerten Verdünnungsmittels durchgeführt werden. Als solche Verdünnungsmittel seien beispielhaft aliphatische Kohlenwasserstoffe wie Pentan, Hexan, Heptan, Cyclohexan, Methylcyclohexan, chlorierte Kohlenwasserstoffe wie Methylenchlorid, Chloroform, 1,2-Dichlorethan, aromatische Kohlenwasserstoffe wie Toluol, Xylol, Mesitylen, chlorierte aromatische Kohlenwasserstoffe wie Chlorbenzol, Dichlorbenzol, Ether wie Diethylether, Methyl-tert-butylether, Tetrahydrofuran, Dioxan, Nitrile wie Acetonitril, Propionitril, Butyronitril, Ester wie Essigsäuremethylester und Essigsäureethylester genannt. Bevorzugt arbeitet man in Methylenchlorid, Chloroform, 1,2-Dichlorethan oder ohne Verdünnungsmittel.

Die Entfernung des Thionylchlorids kann prinzipiell durch Hydrolyse mit Wasser erfolgen. Bevorzugt wird das Thionylchlorid durch Abdestillieren unter vermindertem Druck entfernt.

Das gegebenenfalls vorhandene Verdünnungsmittel wird bevorzugt ebenfalls unter vermindertem Druck abdestilliert.

Im zweiten Schritt des erfindungsgemäßen Verfahrens wird der nach Entfernung des überschüssigen Thionylchlorids und gegebenenfalls des Verdünnungsmittels erhaltene Rückstand in einem neuen Verdünnungsmittel gelöst und durch Erwärmen in diesem Lösungsmittel in die Dithiin-tetracarboximide der Formel (I) überführt. Bevorzugt wird das Reaktionsgemisch hierbei gerührt.

Im zweiten Schritt des erfindungsgemäßen Verfahrens verwendet man organische Lösungsmittel oder Lösungsmittelgemische. Diese Lösungsmittel sind bevorzugt wenigstens teilweise mit Wasser mischbar.

Als Verdünnungsmittel für den zweiten Schritt des erfindungsgemäßen Verfahrens eignen sich im Einzelnen Wasser, Dimethylsulfoxid, Sulfolan, Alkohole wie beispielsweise Methanol, Ethanol, Propanol, Isopropanol, 1- Butanol, 2-Butanol, Isobutanol, Tertiärbutanol, 1-Pentanol, Cyclopentanol, Cyclohexanol, Ethylenglykol, Ethylenglkyol-monomethylether, Kohlenwasserstoffe wie Hexan, Heptan, Cyclohexan, Methylcyclohexan, Toluol, Xylole, Mesitylen, Chlorbenzol, Dichlorbenzol, Nitrobenzol, Ester wie Essigsäuremethylester, Essigsäureethylester, Amide wie Formamid, N,N-Dimethylformamid; N,N-Dimethylacetamid, N-Methylpyrrolidon, Ether wie Methyl-tert-butylether, Tetrahydrofuran, 1,4-Dioxan, Nitrile wie Acetonitril, Propionitril, Butyronitril, Benzonitril, Ketone wie Aceton, Methyl-ethylketon, Methyl-isobutylketon, Pinakolon, Carbonsäuren wie Ameisensäure, Essigsäure, Propionsäure, oder Gemische dieser Verdünnungsmittel.

Bevorzugt verwendet man Wasser, Dimethylsulfoxid, Methanol, Ethanol, Propanol, Isopropanol, 1-Butanol, 2-Butanol, Isobutanol, Tertiärbutanol, 1-Pentanol, Cyclohexanol, Ethylenglykol, Essigsäure-methylester, N,N-Dimethylformamid; N,N-Dimethylacetamid, Tetrahydrofuran, 1,4-Dioxan, Acetonitril, Aceton, Methyl-ethylketon, Methyl-isobutylketon, Essigsäure oder Gemische dieser Verdünnungsmittel.

Ganz besonders bevorzugt verwendet man Gemische aus Wasser und Methanol, Ethanol, Propanol, Isopropanol, 1-Butanol, 2-Butanol, Isobutanol, 1-Pentanol, Essigsäure-methylester, Tetrahydrofuran, 1,4-Dioxan, Acetonitril, Aceton, Essigsäure.

Das Mischungsverhältnis Wasser zu organischem Lösungsmittel kann dabei in weiten Grenzen von beispielsweise 9 zu 1 bis 1 zu 9 variiert werden.

Die Reaktionstemperatur im zweiten Schritt des erfindungsgemäßen Verfahrens kann in weiten Grenzen variiert werden und liegt zwischen 0°C und 200°C. Bevorzugt arbeiten man bei Temperaturen zwischen 20°C und 150°C, besonders bevorzugt zwischen 30°C und 130°C.

Die Reaktionszeit im zweiten Schritt des erfindungsgemäßen Verfahrens liegt zwischen 5 Minuten und 24 Stunden. Bevorzugt arbeitet man zwischen 30 Minuten und 12 Stunden, besonders bevorzugt zwischen 1 und 6 Stunden.

Das erfindungsgemäße Verfahren wird durch folgende Beispiele veranschaulicht, ohne auf sie eingeschränkt zu sein.

### Beispiel 1

Man legt 5,24 g [40 mmol] Bernsteinsäuremethylamid vor und tropft bei 15°C 142,8 g [1200 mmol] Thionylchlorid zu. Es wird dann auf 80°C erwärmt und 1 Stunde bei dieser Temperatur gerührt. Das Reaktionsgemisch wird am Rotationsverdampfer eingeengt. Man erhält 9,9 g eines dicken braunen Öles, das laut HPLC- und LC/MS-Analyse 19,2 Flächen-% an Verbindung (VIII-1), 36,1 Flächen-% an Verbindung (I-1) und 19,1 Flächen-% an Verbindung (IX-1) enthält.
Verbindung (VIII-1) (R = Me):
   LC/MS (ESI neg.): m/z = 256 ([M-H⁺], ³⁵Cl; 65%), 176 ([M-H⁺]-80, ³⁵Cl, 100%).
   ¹³C-NMR (CDCl₃): δ = 25,1 (N-CH₃), 135,5, 138,1 (=C-Cl, =C-S), 162,9, 164,9 (-CO-CS, -CO-C-Cl) ppm.

### Beispiel 2

Zu einer Lösung von 10,5 g [80 mmol] Bernsteinsäuremethylamid in 100 ml Methylenchlorid tropft man bei 15°C 285,6 g [2400 mmol] Thionylchlorid. Man lässt auf Raumtemperatur kommen, erwärmt auf 40°C und rührt 16 Stunden bei dieser Temperatur. Man kühlt auf Raumtemperatur ab und rührt das Reaktionsgemisch in 800 g Eiswasser ein. Nach Stehen über Nacht bei Raumtemperatur trennt man die organische Phase ab, extrahiert die wässrige Phase mit Methylenchlorid, trocknet die vereinigten organischen Phasen und engt dann am Rotationsverdampfer ein. Man erhält 11,8 g eines dicken braunen Öles, das laut HPLC- und LC/MS-Analyse 25,7 Flächen-% an Verbindung (I-1), 22,9 Flächen-% Verbindung (IX-2) und 37,7 Flächen-% an Verbindung (IX-1) enthält.
Verbindung (IX-2):
   LC/MS (ESI pos.): m/z = 353 (MH⁺, 2 x ³⁵Cl).
   ¹³C-NMR (CD₃CN): δ = 25,4 (N-CH₃), 136,1, 139,4 (=C-S, =C-C1), 164,4, 166,2 (-CO-C-Cl, -CO-C-S) ppm.
Verbindung (IX-1):
   LC/MS (ESI pos.): m/z = 385 (MH⁺, 2 x ³⁵Cl).
   ¹³C-NMR (CDCl₃): δ = 25,3 (N-CH₃), 136,1, 140,0 (=C-S, =C-Cl), 164,3, 166,2 (-CO-C-Cl, -CO-C-S) ppm.

### Beispiel 3

0,2 g des Produktgemisches aus Beispiel 2 werden in 10 ml Methanol gelöst und für 4 Stunden auf 60°C erwärmt. Nach Entfernen des Lösungsmittels ist die Zusammensetzung wie folgt: 61,1 Flächen-% Verbindung (I-1), 9,7 Flächen-% Verbindung (IX-2) und 2,5 Flächen-% Verbindung (IX-1).

### Beispiel 4

0,2 g des Produktgemisches aus Beispiel 2 werden in 10 ml Methanol/Wasser (1:1) gelöst und für 4 Stunden auf 60°C erwärmt. Nach Entfernen des Lösungsmittels ist die Zusammensetzung wie folgt: 90,1 Flächen-% Verbindung (I-1), < 0,1 Flächen-% Verbindung (IX-2) und < 0,1 Flächen-% Verbindung (IX-1).

### Beispiel 5

0,2 g des Produktgemisches aus Beispiel 2 werden in 10 ml 1,4-Dioxan/Wasser (1:1) gelöst und für 4 Stunden auf 60°C erwärmt. Nach Entfernen des Lösungsmittels ist die Zusammensetzung wie folgt: 84,6 Flächen-% Verbindung (I-1), < 0,1 Flächen-% Verbindung (IX-2) und < 0,1 Flächen-% Verbindung (IX-1).

### Beispiel 6

0,2 g des Produktgemisches aus Beispiel 2 werden in 10 ml DMF gelöst und für 4 Stunden auf 60°C erwärmt. Nach Entfernen des Lösungsmittels ist die Zusammensetzung wie folgt: 71,1 Flächen-% Verbindung (I-1), < 0,1 Flächen-% Verbindung (IX-2) und < 0,1 Flächen-% Verbindung (IX-1).

### Beispiel 7

0,2 g des Produktgemisches aus Beispiel 2 werden in 10 ml Acetonitril/Wasser (1:1) gelöst und für 4 Stunden auf 60°C erwärmt. Nach Entfernen des Lösungsmittels ist die Zusammensetzung wie folgt: 85,4 Flächen-% Verbindung (I-1), < 0,1 Flächen-% Verbindung (IX-2) und < 0,1 Flächen-% Verbindung (IX-1).

### Beispiel 8

0,2 g des Produktgemisches aus Beispiel 2 werden in 10 ml Aceton/Wasser (1:1) gelöst und für 4 Stunden auf 60°C erwärmt. Nach Entfernen des Lösungsmittels ist die Zusammensetzung wie folgt: 85,1 Flächen-% Verbindung (I-1), < 0,1 Flächen-% Verbindung (IX-2) und < 0,1 Flächen-% Verbindung (IX-1).

### Beispiel 9

0,2 g des Produktgemisches aus Beispiel 2 werden in 10 ml Essigsäure-methylester/Wasser (1:1) gelöst und für 4 Stunden auf 60°C erwärmt. Nach Entfernen des Lösungsmittels ist die Zusammensetzung wie folgt: 89 Flächen-% Verbindung (I-1), < 0,1 Flächen-% Verbindung (IX-2) und < 0,1 Flächen-% Verbindung (IX-1).

### Beispiel 10

Zu einer Lösung von 5,24 g [40 mmol] Bernsteinsäuremethylamid in 50 ml Methylenchlorid tropft man bei 15°C 142,8 g [1200 mmol] Thionylchlorid. Man lässt auf Raumtemperatur kommen, erwärmt auf 40°C und rührt 1 Stunde bei dieser Temperatur. Man kühlt auf Raumtemperatur ab und engt am Rotationsverdampfer ein. Man erhält 12 g eines dicken braunen Öles, welches man in 100 ml Ethanol löst. Diese Lösung wird 4 Stunden auf 60°C erwärmt und anschließend auf Raumtemperatur abgekühlt. Der ausgefallene dunkelgrüne Feststoff wird abgesaugt, mit EtOH und Wasser gewaschen und getrocknet. Es resultieren 2,92 g Feststoff, der laut HPLC-Analyse zu 99,1 Flächen-% aus der Verbindung (I-1) besteht, was einer Ausbeute von 51,3% der Theorie entspricht.

### Beispiel 11

Zu einer Lösung von 5,24 g [40 mmol] Bernsteinsäure-methylamid in 50 ml 1,4-Dioxan tropft man bei 15°C 142,8 g [1200 mmol] Thionylchlorid. Man lässt auf Raumtemperatur kommen, erwärmt dann auf 80°C und rührt 1 Stunde bei dieser Temperatur. Man kühlt auf Raumtemperatur ab und engt am Rotationsverdampfer ein. Man erhält 10,9 g eines dicken braunen Öles, welches man in 100 ml Ethanol löst. Diese Lösung wird 4 Stunden auf 60°C erwärmt und anschließend auf Raumtemperatur abgekühlt. Der ausgefallene dunkelgrüne Feststoff wird abgesaugt, mit EtOH und Wasser gewaschen und getrocknet. Es resultieren 2,66 g Feststoff, der lt. HPLC-Analyse zu 99,4 Flächen-% aus der Verbindung (I-1) besteht, was einer Ausbeute von 46,8% der Theorie entspricht.

### Beispiel 12

Man legt 5,24 g [40 mmol] Bernsteinsäuremethylamid vor und tropft bei 15°C 142,8 g [1200 mmol] Thionylchlorid zu. Es wird dann auf 80°C erwärmt und 1 Stunde bei dieser Temperatur gerührt. Das Reaktionsgemisch wird am Rotationsverdampfer eingeengt. Der Rückstand (dunkelbraunes dickes Öl) wird mit 100 ml Methanol/Wasser (1:1) versetzt und 4 Stunden auf 60°C erwärmt. Danach lässt man auf Raumtemperatur abkühlen, saugt den ausgefallenen Feststoff ab und wäscht ihn mit Wasser und Methanol. Nach Trocknen resultieren 4,30 g dunkelgrüner Feststoff, der laut HPLC-Analyse zu 94,7 Flächen-% aus der Verbindung (I-1) besteht, was einer Ausbeute von 72,1% der Theorie entspricht.

### Beispiel 13

Man legt 5,24 g [40 mmol] Bernsteinsäuremethylamid vor und tropft bei 15°C 47,6 g [400 mmol] Thionylchlorid zu. Es wird dann auf 80°C erwärmt und 1 Stunde bei dieser Temperatur gerührt. Das Reaktionsgemisch wird am Rotationsverdampfer eingeengt. Der Rückstand (dunkelbraunes dickes Öl) wird mit 100 ml Methanol/Wasser (1:1) versetzt und 4 Stunden auf 60°C erwärmt. Danach lässt man auf Raumtemperatur abkühlen, saugt den ausgefallenen Feststoff ab und wäscht ihn mit Wasser und Methanol. Nach Trocknen resultieren 4,05 g dunkelgrüner Feststoff, der lt. HPLC-Analyse zu 97,8 Flächen-% aus der Verbindung (I-1) besteht, was einer Ausbeute von 70,2% der Theorie entspricht.

### Vergleichsbeispiel 1

Zu einer Lösung von 5,24 g [40 mmol] Bernsteinsäuremethylamid in 50 ml 1,4-Dioxan tropft man bei 15°C 142,8 g [1200 mmol] Thionylchlorid. Man lässt auf Raumtemperatur kommen und rührt 16 Stunden bei Raumtemperatur. Das Reaktionsgemisch wird in ca. 400 g Eiswasser eingerührt. Man setzt ca. 100 ml Essigsäureethylester zu und saugt den ausgefallenen grünen Feststoff ab, wäscht mit Wasser und Essigsäureethylester und trocknet. Es resultieren 1,00 g Feststoff einer Reinheit von 80,8 Flächen-%, was einer Ausbeute von 14,3% der Theorie entspricht. Die organische Phase des Filtrates wird abgetrennt und mit Wasser gewaschen. Dadurch fällt an der Phasengrenzfläche weiterer Feststoff aus: 0,40 g, 99,1%ig (7,2% der Theorie). Das Filtrat der organischen Phase wird eingeengt und der Rückstand mit Methyl-tertiärbutylether (MTBE) verrührt. Der so erhaltenen Feststoff wird abgesaugt und getrocknet: 0,70 g, 51,0%ig (6,3% der Theorie). Die gesammelten wässrigen Phasen lässt man 2 Tage bei Raumtemperatur stehen; danach ist weiterer Feststoff ausgefallen, der ebenfalls isoliert wird: 0,50 g, 99,1%ig (8,8% der Theorie). Insgesamt beträgt die erhaltene Ausbeute also 36,5% der Theorie an Verbindung (I-1).

### Vergleichsbeispiel 2

Zu einer Lösung von 5,24 g [40 mmol] Bernsteinsäuremethylamid in 50 ml 1,4-Dioxan tropft man bei 15°C 142,8 g [1200 mmol] Thionylchlorid. Man lässt auf Raumtemperatur kommen, erwärmt dann auf 80°C und rührt 1 Stunde bei dieser Temperatur. Man kühlt auf Raumtemperatur ab, rührt in ca. 400 g Eiswasser ein, lässt über Nacht stehen, saugt den ausgefallenen grünen Feststoff ab, wäscht mit Wasser und Essigester und trocknet. Es resultieren 3,75 g Feststoff einer Reinheit von nur 76,5 Flächen-% (50,8% der Theorie).

### Beispiel 14

Man legt 6,93 g [40 mmol] Bernsteinsäuretertiärbutylamid vor und tropft bei 15°C 142,8 g [1200 mmol] Thionylchlorid zu. Es wird dann auf 80°C erwärmt und 1 Stunde bei dieser Temperatur gerührt. Das Reaktionsgemisch wird am Rotationsverdampfer eingeengt. Das resultierende dicke braune Öl wird in Methylenchlorid gelöst und mit gesättigter wässriger NaCl-Lösung gewaschen. Die organische Phase wird über Natriumsulfat getrocknet und eingeengt. Man erhält 7,65 g braunen Rückstand, der laut HPLC und LC/MS 14,3 Flächen-% an Verbindung (IX-3) und 9,7 Flächen-% Verbindung (IX-4) enthält.
Verbindung (IX-3):
   LC/MS (ESI pos.): m/z = 437 ([MH⁺], 2 x ³⁵Cl; 60%), 454 ([MH⁺] + NH₃, 2x ³⁵Cl, 100%).
Verbindung (IX-4):
   LC/MS (ESI pos.): m/z = 469 ([MH⁺], 2 x ³⁵Cl; 20%), 486 ([MH⁺] + NH₃, 2x ³⁵Cl, 100%).

### Beispiel 15

Man legt 6,93 g [40 mmol] Bernsteinsäuretertiärbutylamid vor und tropft bei 15°C 142,8 g [1200 mmol] Thionylchlorid zu. Es wird dann auf 50°C erwärmt und 4 Stunden bei dieser Temperatur gerührt. Das Reaktionsgemisch wird am Rotationsverdampfer eingeengt. Das resultierende dicke braune Öl wird in 100 ml EtOH/H₂O (1:1) aufgenommen und 4 Stunden auf 60°C erhitzt. Danach wird auf Raumtemperatur abgekühlt, der Feststoff abgesaugt, mit Wasser und EtOH gewaschen und getrocknet. Man erhält 5,00 g braunen Feststoff, der laut HPLC 99,6 Flächen-% Verbindung (I-3) enthält.

### Beispiel 16

Man legt 8 g [40 mmol] Bernsteinsäurecyclohexylamid vor und tropft bei 15°C 142,8 g [1200 mmol] Thionylchlorid zu. Es wird dann auf 80°C erwärmt und 1 Stunde bei dieser Temperatur gerührt. Das Reaktionsgemisch wird am Rotationsverdampfer eingeengt. Das resultierende dicke braune Öl wird in Methylenchlorid gelöst und mit gesättigter wässriger NaCl-Lösung gewaschen. Die organische Phase wird über Natriumsulfat getrocknet und eingeengt. Man erhält 10,8 g braunen Rückstand, der laut HPLC und LC/MS 19 Flächen-% an Verbindung (IX-5) enthält.
Verbindung (IX-5):
   LC/MS (ESI pos.): m/z = 521 ([MH⁺], 2 x ³⁵Cl; 70%), 538 ([MH⁺] + NH₃, 2 x ³⁵Cl, 100%).

### Beispiel 17

Man legt 8 g [40 mmol] Bernsteinsäurecyclohexylamid vor und tropft bei 15°C 142,8 g [1200 mmol] Thionylchlorid zu. Es wird dann auf 50°C erwärmt und 4 Stunden bei dieser Temperatur gerührt. Das Reaktionsgemisch wird am Rotationsverdampfer eingeengt. Das resultierende dicke braune Öl wird in 100 ml EtOH/H₂O (1:1) aufgenommen und 4 Stunden auf 60°C erhitzt. Danach wird auf Raumtemperatur abgekühlt, der Feststoff abgesaugt, mit Wasser und EtOH gewaschen und getrocknet. Man erhält 2,86 g braunen Feststoff, der laut HPLC 92,1 Flächen-% Verbindung (I-3) enthält.

### Beispiel 18

Man legt 6,37 g [40 mmol] Bernsteinsäurepropylamid vor und tropft bei 15°C 142,8 g [1200 mmol] Thionylchlorid zu. Es wird dann auf 50°C erwärmt und 4 Stunden bei dieser Temperatur gerührt. Das Reaktionsgemisch wird am Rotationsverdampfer eingeengt. Das resultierende dicke braune Öl wird in 100 ml EtOH/H₂O (1:1) aufgenommen und 4 Stunden auf 60°C erhitzt. Danach wird auf Raumtemperatur abgekühlt, der Feststoff abgesaugt, mit Wasser und EtOH gewaschen und getrocknet. Man erhält 2,15 g grünen Feststoff, der laut HPLC 99,4 Flächen-% Verbindung (I-4) enthält.

### Allgemeine Angaben:

HPLC-Bedingungen: Zorbax Eclipse Plus C18 4.6*50 mm 1.8µm, Eluent A: 0.1% H₃P0₄, Eluent B: Acetonitril, Gradient: 90/10, 20%/min, 5/95 (1.75), Fluss: 2 ml/min, 55°C.

## Patentansprüche

1. Verfahren zum Herstellen von Dithiin-tetracarboximide der allgemeinen Formel (I) 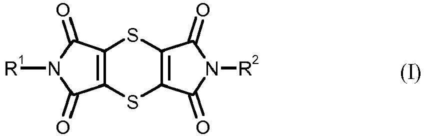 in welcher
R¹ und R² gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
R³ für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
R⁴ für Hydroxy, C₁-C₄-Alkyl oder C₁-C₄-Alkoxy steht,
**dadurch gekennzeichnet, dass** man
in einer ersten Stufe Bernsteinsäuremonoamide der Formel (VI) 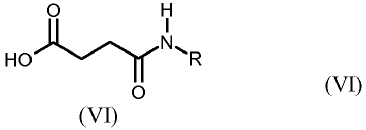 in welcher R für R¹ oder R² steht,
mit einem Überschuss an Thionylchlorid gegebenenfalls in Gegenwart eines Verdünnungsmittels umsetzt,
anschließend den Überschuss an Thionylchlorid entfernt und das so erhaltene Produktgemisch in einer zweiten Stufe in einem organischen Lösungsmittel in die Dithiin-tetracarboximide der Formel (I) überführt.

2. Verfahren gemäß Anspruch 1, **dadurch gekennzeichnet, dass** man im ersten Schritt zwischen 2 und 100 Mol Thionylchlorid pro Mol Bernsteinsäuremonoamid der Formel (VI) einsetzt.

3. Verfahren gemäß Anspruch 1 oder 2, **dadurch gekennzeichnet, dass** der erste Schritt ohne Verdünnungsmittel durchgeführt wird.

4. Verfahren gemäß Anspruch 1, 2 oder 3, **dadurch gekennzeichnet, dass** man im zweiten Schritt ein organisches Lösungsmittel einsetzt, welches wenigstens teilweise mit Wasser mischbar ist.

5. Verfahren gemäß Anspruch 1, 2, 3 oder 4, **dadurch gekennzeichnet, dass** man im zweiten Schritt als Lösungsmittel Wasser, Dimethylsulfoxid, Sulfolan, Alkohole wie Methanol, Ethanol, Propanol, Isopropanol, 1- Butanol, 2-Butanol, Isobutanol, Tertiärbutanol, 1-Pentanol, Cyclopentanol, Cyclohexanol, Ethylenglykol, Ethylenglkyol-monomethylether, Kohlenwasserstoffe wie Hexan, Heptan, Cyclohexan, Methylcyclohexan, Toluol, Xylole, Mesitylen, Chlorbenzol, Dichlorbenzol, Nitrobenzol, Ester wie Essigsäuremethylester, Essigsäureethylester, Amide wie Formamid, N,N-Dimethylformamid; N,N-Dimethylacetamid, N-Methylpyrrolidon, Ether wie Methyl-tert-butylether, Tetrahydrofuran, 1,4-Dioxan, Nitrile wie Acetonitril, Propionitril, Butyronitril, Benzonitril, Ketone wie Aceton, Methyl-ethylketon, Methyl-isobutylketon, Pinakolon, Carbonsäuren wie Ameisensäure, Essigsäure, Propionsäure oder Gemische dieser Verdünnungsmittel einsetzt.

6. Verfahren gemäß Anspruch 1, 2, 3, 4 oder 5, **dadurch gekennzeichnet, dass** man den zweiten Schritt unter Erwärmung durchführt.

7. Verfahren zum Herstellen von Polysulfiden der Formel (IX), 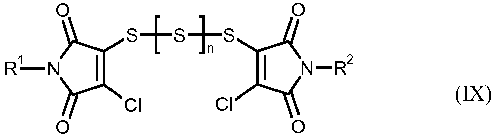 in welcher
R¹ und R² gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
R³ für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
R⁴ für Hydroxy, C₁-C₄-Alkyl oder C₁-C₄-Alkoxy steht,
n für 0, 1 oder 2 steht,
sowie von Thiosulfonsäurederivaten der Formel (VIII) 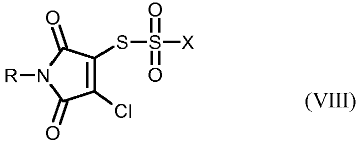 in welcher R für R¹ oder R² steht und X für Chlor oder Hydroxy steht, **dadurch gekennzeichnet, dass** man
in einer ersten Stufe Bernsteinsäuremonoamide der Formel (VI) 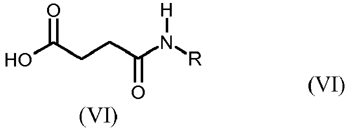 in welcher R für R¹ oder R² steht,
mit einem Überschuss an Thionylchlorid gegebenenfalls in Gegenwart eines Verdünnungsmittels umsetzt.

8. Polysulfide der Formel (IX), 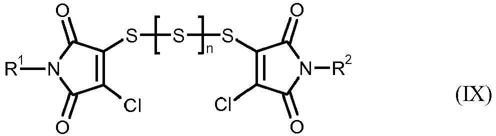 in welcher
R¹ und R² gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
R³ für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
R⁴ für Hydroxy, C₁-C₄-Alkyl oder C₁-C₄-Alkoxy steht,
n für 0, 1 oder 2 steht.

9. Thiosulfonsäurederivate der allgemeinen Formel (VIII) 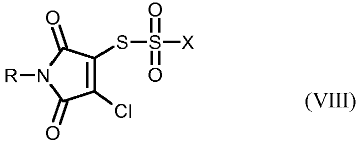 in welcher
R für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) steht,
R³ für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
R⁴ für Hydroxy, C₁-C₄-Alkyl oder C₁-C₄-Alkoxy steht;
X für Chlor oder Hydroxy steht.

## Claims

1. Process for preparing dithiine-tetracarboximides of the general formula (I) 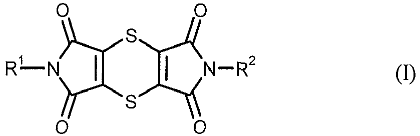 in which
R¹ and R² are identical or different and are hydrogen, or are C₁-C₈-alkyl which is optionally substituted one or more times by halogen, -OR³, and/or -COR⁴, are C₃-C₇-cycloalkyl which is optionally substituted one or more times by halogen, C₁-C₄-alkyl or C₁-C₄-haloalkyl, or are aryl or aryl-(C₁-C₄-alkyl) each of which is optionally substituted one or more times by halogen, C₁-C₄-alkyl, C₁-C₄-haloalkyl, -COR⁴ or sulphonylamino,
R³ is hydrogen, C₁-C₄-alkyl or C₁-C₄-alkylcarbonyl or is aryl which is optionally substituted one or more times by halogen, C₁-C₄-alkyl or C₁-C₄-haloalkyl,
R⁴ is hydroxyl, C₁-C₄-alkyl or C₁-C₄-alkoxy,
**characterized in that**
in a first stage, succinic monoamides of the formula (VI) 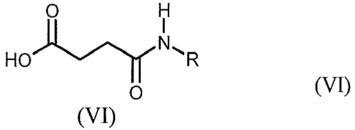 in which R is R¹ or R²
are reacted with an excess of thionyl chloride, optionally in the presence of a diluent,
then the excess of thionyl chloride is removed and the resulting product mixture is converted in a second stage, in an organic solvent, into the dithiine-tetracarboximides of the formula (I).

2. Process according to Claim 1, **characterized in that** in the first step between 2 and 100 mol of thionyl chloride are used per mole of succinic monoamide of the formula (VI).

3. Process according to Claim 1 or 2, **characterized in that** the first step is carried out without diluent.

4. Process according to Claim 1, 2 or 3, **characterized in that** in the second step an organic solvent is used which is at least partly miscible with water.

5. Process according to Claim 1, 2, 3 or 4, **characterized in that** solvent used in the second step comprises water, dimethyl sulphoxide, sulpholane, alcohols such as methanol, ethanol, propanol, isopropanol, 1-butanol, 2-butanol, isobutanol, tertiary-butanol, 1-pentanol, cyclopentanol, cyclohexanol, ethylene glycol, ethylene glycol monomethyl ether, hydrocarbons such as hexane, heptane, cyclohexane, methylcyclohexane, toluene, xylenes, mesitylene, chlorobenzene, dichlorobenzene, nitrobenzene, esters such as methyl acetate, ethyl acetate, amides such as formamide, N,N-dimethylformamide; N,N-dimethylacetamide, N-methylpyrrolidone, ethers such as methyl tert-butyl ether, tetrahydrofuran, 1,4-dioxane, nitriles such as acetonitrile, propionitrile, butyronitrile, benzonitrile, ketones such as acetone, methyl ethyl ketone, methyl isobutyl ketone, pinacolone, carboxylic acids such as formic acid, acetic acid, propionic acid, or mixtures of these diluents.

6. Process according to Claim 1, 2, 3, 4 or 5, **characterized in that** the second step is carried out with heating.

7. Process for preparing polysulphides of the formula (IX), 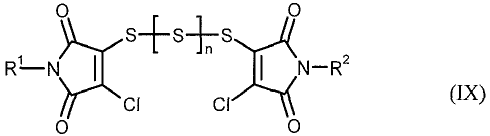 in which
R¹ and R² are identical or different and are hydrogen, or are C₁-C₈-alkyl which is optionally substituted one or more times by halogen, -OR³, and/or -COR⁴, are C₃-C₇-cycloalkyl which is optionally substituted one or more times by halogen, C₁-C₄-alkyl or C₁-C₄-haloalkyl, or are aryl or aryl-(C₁-C₄-alkyl) each of which is optionally substituted one or more times by halogen, C₁-C₄-alkyl, C₁-C₄-haloalkyl, -COR⁴ or sulphonylamino,
R³ is hydrogen, C₁-C₄-alkyl or C₁-C₄-alkylcarbonyl or is aryl which is optionally substituted one or more times by halogen, C₁-C₄-alkyl or C₁-C₄-haloalkyl,
R⁴ is hydroxyl, C₁-C₄-alkyl or C₁-C₄-alkoxy,
n is 0, 1 or 2,
and also for preparing thiosulphonic acid derivatives of the formula (VIII) 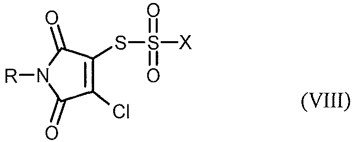 in R is R¹ or R² and X is chlorine or hydroxyl,
**characterized in that**
in a first stage, succinic monoamides of the formula (VI) 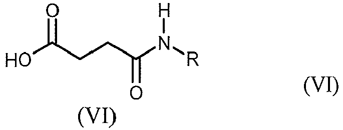 in which R is R¹ or R²
are reacted with an excess of thionyl chloride, optionally in the presence of a diluent.

8. Polysulphides of the formula (IX), 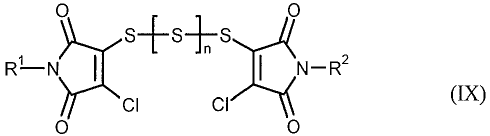 in which
R¹ and R² are identical or different and are hydrogen, or are C₁-C₈-alkyl which is optionally substituted one or more times by halogen, -OR³, and/or -COR⁴, are C₃-C₇-cycloalkyl which is optionally substituted one or more times by halogen, C₁-C₄-alkyl or C₁-C₄-haloalkyl, or are aryl or aryl-(C₁-C₄-alkyl) each of which is optionally substituted one or more times by halogen, C₁-C₄-alkyl, C₁-C₄-haloalkyl, -COR⁴ or sulphonylamino,
R³ is hydrogen, C₁-C₄-alkyl or C₁-C₄-alkylcarbonyl or is aryl which is optionally substituted one or more times by halogen, C₁-C₄-alkyl or C₁-C₄-haloalkyl,
R⁴ is hydroxyl, C₁-C₄-alkyl or C₁-C₄-alkoxy,
n is 0, 1 or 2.

9. Thiosulphonic acid derivatives of the general formula (VIII) 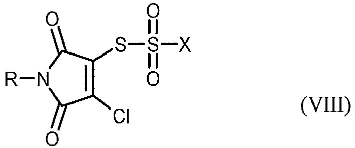 in which
R is hydrogen, or is C₁-C₈-alkyl which is optionally substituted one or more times by halogen, -OR³, and/or -COR⁴, are C₃-C₇-cycloalkyl which is optionally substituted one or more times by halogen, C₁-C₄-alkyl or C₁-C₄-haloalkyl, or is aryl or aryl-(C₁-C₄-alkyl) each of which is optionally substituted one or more times by halogen, C₁-C₄-alkyl, C₁-C₄-haloalkyl, -COR⁴ or sulphonylamino,
R³ is hydrogen, C₁-C₄-alkyl or C₁-C₄-alkylcarbonyl or is aryl which is optionally substituted one or more times by halogen, C₁-C₄-alkyl or C₁-C₄-haloalkyl,
R⁴ is hydroxyl, C₁-C₄-alkyl or C₁-C₄-alkoxy;
X is chlorine or hydroxyl.

## Revendications

1. Procédé de fabrication de dithiine-tétracarboximides de formule générale (I) 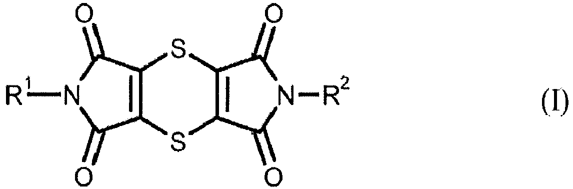 dans laquelle
R¹ et R² sont identiques ou différents, et représentent hydrogène, alkyle en C₁-C₈ éventuellement substitué une ou plusieurs fois par halogène, -OR³, -COR⁴; cycloalkyle en C₃-C₇ éventuellement substitué une ou plusieurs fois par halogène, alkyle en C₁-C₄ ou halogénoalkyle en C₁-C₄ ; aryle ou aryl- (alkyle en C₁-C₄), chacun éventuellement substitué une ou plusieurs fois par halogène, alkyle en C₁-C₄, halogénoalkyle en C₁-C₄, -COR⁴ ou sulfonylamino,
R³ représente hydrogène, alkyle en C₁-C₄, alkylcarbonyle en C₁-C₄ ou aryle éventuellement substitué une ou plusieurs fois par halogène, alkyle en C₁-C₄ ou halogénoalkyle en C₁-C₄,
R⁴ représente hydroxy, alkyle en C₁-C₄ ou alcoxy en C₁-C₄,
**caractérisé en ce que**
lors d'une première étape, des monoamides de l'acide succinique de formule (VI) 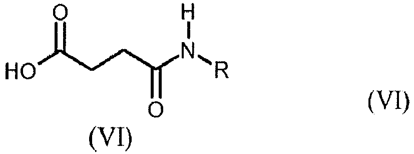 dans laquelle R représente R¹ ou R²,
sont mis en réaction avec un excès de chlorure de thionyle, éventuellement en présence d'un diluant,
puis l'excès de chlorure de thionyle est éliminé et le mélange de produits ainsi obtenu est transformé lors d'une seconde étape dans un solvant organique en les dithiine-tétracarboximides de formule (I).

2. Procédé selon la revendication 1, **caractérisé en ce que**, lors de la première étape, entre 2 et 100 moles de chlorure de thionyle par mole de monoamide de l'acide succinique de formule (VI) sont utilisées.

3. Procédé selon la revendication 1 ou 2, **caractérisé en ce que** la première étape est réalisée sans diluant.

4. Procédé selon la revendication 1, 2 ou 3, **caractérisé en ce que**, lors de la seconde étape, un solvant organique qui est au moins partiellement miscible avec l'eau est utilisé.

5. Procédé selon la revendication 1, 2, 3 ou 4, **caractérisé en ce que**, lors de la seconde étape, de l'eau, du diméthylsulfoxyde, du sulfolane, des alcools tels que le méthanol, l'éthanol, le propanol, l'isopropanol, le 1-butanol, le 2-butanol, l'isobutanol, le tert.-butanol, le 1-pentanol, le cyclopentanol, le cyclohexanol, l'éthylène glycol, l'éther monométhylique d'éthylène glycol, des hydrocarbures tels que l'hexane, l'heptane, le cyclohexane, le méthylcyclohexane, le toluène, les xylènes, le mésitylène, le chlorobenzène, le dichlorobenzène, le nitrobenzène, des esters tels que l'ester méthylique de l'acide acétique, l'ester éthylique de l'acide acétique, des amides tels que le formamide, le N,N-diméthylformamide, le N,N-diméthylacétamide, la N-méthylpyrrolidone, des éthers tels que l'éther de méthyle et de tert.-butyle, le tétrahydrofurane, le 1,4-dioxane, des nitriles tels que l'acétonitrile, le propionitrile, le butyronitrile, le benzonitrile, des cétones telles que l'acétone, la méthyléthylcétone, la méthylisobutylcétone, la pinacolone, des acides carboxyliques tels que l'acide formique, l'acide acétique, l'acide propionique ou des mélanges de ces diluants sont utilisés en tant que solvant.

6. Procédé selon la revendication 1, 2, 3, 4 ou 5, **caractérisé en ce que** la seconde étape est réalisée avec chauffage.

7. Procédé de fabrication de polysulfures de formule (IX) 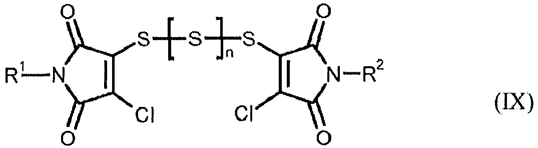 dans laquelle
R¹ et R² sont identiques ou différents, et représentent hydrogène, alkyle en C₁-C₈ éventuellement substitué une ou plusieurs fois par halogène, -OR³, -COR⁴; cycloalkyle en C₃-C₇ éventuellement substitué une ou plusieurs fois par halogène, alkyle en C₁-C₄ ou halogénoalkyle en C₁-C₄ ; aryle ou aryl- (alkyle en C₁-C₄), chacun éventuellement substitué une ou plusieurs fois par halogène, alkyle en C₁-C₄, halogénoalkyle en C₁-C₄, -COR⁴ ou sulfonylamino,
R³ représente hydrogène, alkyle en C₁-C₄, alkylcarbonyle en C₁-C₄ ou aryle éventuellement substitué une ou plusieurs fois par halogène, alkyle en C₁-C₄ ou halogénoalkyle en C₁-C₄,
R⁴ représente hydroxy, alkyle en C₁-C₄ ou alcoxy en C₁-C₄,
n représente 0, 1 ou 2,
ainsi que de dérivés de l'acide thiosulfonique de formule (VIII) 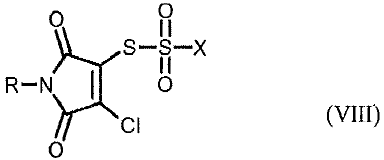 dans laquelle R représente R¹ ou R² et X représente chlore ou hydroxy,
**caractérisé en ce que**
lors d'une première étape, des monoamides de l'acide succinique de formule (VI) 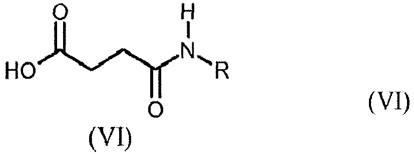 dans laquelle R représente R¹ ou R²,
sont mis en réaction avec un excès de chlorure de thionyle, éventuellement en présence d'un diluant.

8. Polysulfures de formule (IX) 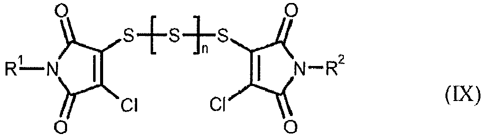 dans laquelle
R¹ et R² sont identiques ou différents, et représentent hydrogène, alkyle en C₁-C₈ éventuellement substitué une ou plusieurs fois par halogène, -OR³, -COR⁴ ; cycloalkyle en C₃-C₇ éventuellement substitué une ou plusieurs fois par halogène, alkyle en C₁-C₄ ou halogénoalkyle en C₁-C₄ ; aryle ou aryl- (alkyle en C₁-C₄), chacun éventuellement substitué une ou plusieurs fois par halogène, alkyle en C₁-C₄, halogénoalkyle en C₁-C₄, -COR⁴ ou sulfonylamino,
R³ représente hydrogène, alkyle en C₁-C₄, alkylcarbonyle en C₁-C₄ ou aryle éventuellement substitué une ou plusieurs fois par halogène, alkyle en C₁-C₄ ou halogénoalkyle en C₁-C₄,
R⁴ représente hydroxy, alkyle en C₁-C₄ ou alcoxy en C₁-C₄,
n représente 0, 1 ou 2,

9. Dérivés de l'acide thiosulfonique de formule générale (VIII) 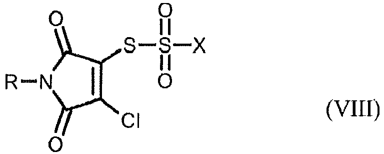 dans laquelle
R représente hydrogène, alkyle en C₁-C₈ éventuellement substitué une ou plusieurs fois par halogène, -OR³, -COR⁴ ; cycloalkyle en C₃-C₇ éventuellement substitué une ou plusieurs fois par halogène, alkyle en C₁-C₄ ou halogénoalkyle en C₁-C₄ ; aryle ou aryl-(alkyle en C₁-C₄), chacun éventuellement substitué une ou plusieurs fois par halogène, alkyle en C₁-C₄, halogénoalkyle en C₁-C₄, -COR⁴ ou sulfonylamino,
R³ représente hydrogène, alkyle en C₁-C₄, alkylcarbonyle en C₁-C₄ ou aryle éventuellement substitué une ou plusieurs fois par halogène, alkyle en C₁-C₄ ou halogénoalkyle en C₁-C₄,
R⁴ représente hydroxy, alkyle en C₁-C₄ ou alcoxy en C₁-C₄,
X représente chlore ou hydroxy.
